Europäisches Patentamt

European Patent Office

Office européen des brevets

(19)

(11) Publication number: **0 347 224**
**A2**

## (12) EUROPEAN PATENT APPLICATION

(21) Application number: **89306071.5**

(22) Date of filing: **15.06.89**

(51) Int. Cl.⁴: **C 12 N 15/00**
**C 12 N 1/20, C 12 N 5/00,**
**C 12 N 9/08**

(30) Priority: **15.06.88 JP 147884/88**

(43) Date of publication of application:
**20.12.89 Bulletin 89/51**

(84) Designated Contracting States:
**BE CH DE ES FR GB IT LI NL SE**

(71) Applicant: **TOYO JOZO KABUSHIKI KAISHA**
**632-1 Mifuku Ohito-cho**
**Tagata-gun Shizuoka-ken (JP)**

(72) Inventor: **Akasaka, Masami**
**632-1 Mifuku Ohito-cho**
**Tagata-gun Shizuoka (JP)**

**Kubota, Akiko**
**9-2 Tokiwa-cho SEnbon**
**Numazu-shi Shizuoka (JP)**

**Mizoguchi, Junzo**
**357-1 Onarimon Ohito-cho**
**Tagata-gun Shizuoka (JP)**

**Satoh, Sakae**
**1012-17 Kashiya Kannami-cho**
**Tagata-gun Shizuoka (JP)**

(74) Representative: **Woods, Geoffrey Corlett et al**
**J.A. KEMP & CO. 14 South Square Gray's Inn**
**London WC1R 5EU (GB)**

The microorganism(s) has (have) been deposited with Fermentation Research Institute under number(s) FERM BP-1903.

(54) **Novel glutathione peroxidase gene.**

(57) The present invention provides a DNA sequence which codes for a glutathione peroxidase, the glutathione peroxidase having the amino acid sequence shown in Figure 1, wherein "sec" is selenocystein. The DNA sequence may be incorporated in a vector and expressed in bacterial or mammalian cells.

FIG. I

$X_1$ Ala Phe Ile Ala Lys Ser Phe Tyr Asp
Leu Ser Ala Ile Ser Leu Asp Gly Glu Lys
Val Asp Phe Asn Thr Phe Arg Gly Arg Ala
Val Leu Ile Glu Asn Val Arg Ser Leu Sec
Gly Thr Thr Thr Arg Asp Phe Thr Gln Leu
Asn Glu Leu Gln Cys Arg Phe Pro Arg Arg
Leu Val Val Leu Gly Phe Pro Cys Asn Gln
Phe Gly His Gln Glu Asn Cys Gln Asn Glu
Glu Ile Leu Asn Ser Leu Lys Tyr Val Arg
Pro Gly Gly Gly Tyr Gln Pro Thr Phe Thr
Leu Val Gln Lys Cys Glu Val Asn Gly Gln
Asn Glu His Pro Val Phe Ala Tyr Leu Lys
Asp Lys Leu Pro Tyr Pro Tyr Asp Asp Pro
Phe Ser Leu Met Thr Asp Pro Lys Leu Ile
Ile Trp Ser Pro Val Arg Arg Ser Asp Val
Ala Trp Asn Phe Glu Lys Phe Leu Ile Gly
Pro Glu Gly Glu Pro Phe Arg Arg Tyr Ser
Arg Thr Phe Pro Thr Ile Asn Ile Glu Pro
Asp Ile Lys Arg Leu Leu Lys Val Ala Ile

**Description**

## NOVEL GLUTATHIONE PEROXIDASE GENE

This invention relates to a novel deoxyribonucleic acid having genetic information of glutathione peroxidase, a transformant containing the said DNA, and polypeptide obtained by expressing the said genetic information of the said DNA with said transformant and its production.

[Prior arts]

Glutathione peroxidase is an enzyme which catalyses a reaction of two moles of glutathione and one mole of hydrogen peroxide to form two moles of glutathione-oxide and two moles of water, and has been known to distribute in mammalian tissues and organs such as liver, kidney, heart, lung, red blood cell and blood plasma. [Flohe, L. et al., FEBS Lett., 32 : 132-134 (1973) ] It provides an important role for treatment of biological peroxide by catalyzing two electrons' reduction of lipid-peroxide with glutathione. Glutathione peroxidase is a protein containing selenium which locates in the form of seleno-cystein (Sec) in its active center. According to a study on cloned mouse glutathione peroxidase gene, an opal codon TGA of deoxyribonucleic acid sequence (DNA), which is in general read to a termination codon, in this enzyme is coding a seleno-cystein (Sec) [EMBO J., Vol. 5, No. 6, pp 1221-1227 (1986) ].

A human type glutathione peroxidase (hereinafter sometimes designates as h-GSHPx) has been separated from erythrocyte and blood plasma, and has been known to be a homotetramer, in which a molecular weight of erythrocyte type subunit thereof is 20,600 and that of blood plasma type subunit is 21,500. [Archivs of Biochemistry and Biophysics, Vol. 256, (2) : 677-686 (1987) and The Journal of Biological Chemistry, Vol. 262 (36) : pp 17398-17403 (1987)].

An h-GSHPx of erythrocytes and that of liver and kidney are estimated as an identical substance due to crossing immunologically and has molecular weight of their subunit of 20600. The h-GSHPx gene is quite homologous to mouse GSHPx gene, however these are clearly different from h-GSHPx of blood plasma in immunologically. Hence at present more than two kinds of h-GSHPx have been known.

These h-GSHPx were cloned using c-DNA library of m-RNA from liver and kidney cells, and their gene structure has been determined. [Nucleic Acids Research, Vol. 15, No.13, pp 5484 (1987), ibid., Vol. 15, No.17, pp 7178 (1987) ]. Though h-GSHPx protein of blood plasma has been isolated, its gene has not been cloned.

BACKGROUND OF THE INVENTION :

The h-GSHPx gene of liver and kidney cells has been cloned and its structure has also been elucidated. We have successfully obtained the novel h-GSHPx gene by cloning the different type of h-GSHPx gene using c-DNA library of human liver, and elucidated its structure.

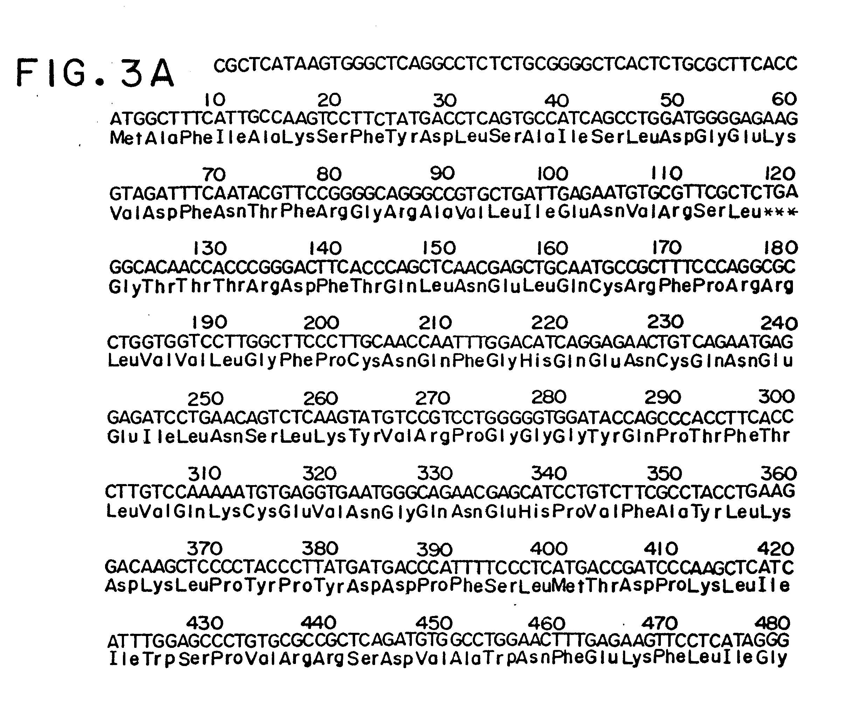

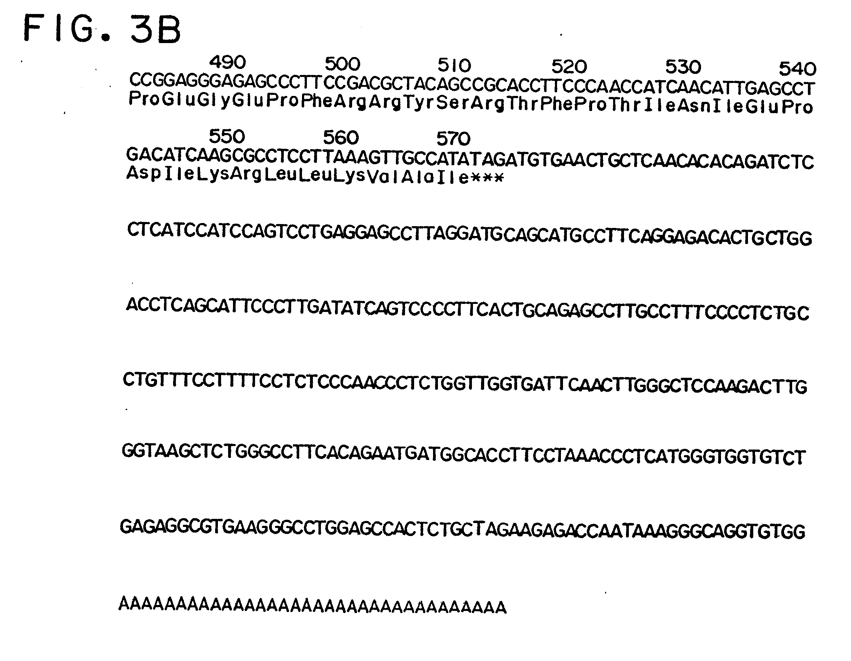

We have made cloning the gene of 951 base pairs extended from poly-A tail in Figs. 3A and 3B using c-DNA library of human liver. This clone has open reading frame containing a sequence from an initiation coden to termination codon, and at least has a polypeptide structure of h-GSHPx comprising 189 amino acids of the formula,

```
 Ala - Phe - Ile - Ala - Lys
- Ser - Phe - Tyr - Asp - Leu
- Ser - Ala - Ile - Ser - Leu
- Asp - Gly - Glu - Lys - Val
- Asp - Phe - Asn - Thr - Phe
- Arg - Gly - Arg - Ala - Val
- Leu - Ile - Glu - Asn - Val
- Arg - Ser - Leu - *** - Gly
- Thr - Thr - Thr - Arg - Asp
- Phe - Thr - Gln - Leu - Asn
- Glu - Leu - Gln - Cys - Arg
- Phe - Pro - Arg - Arg - Leu
```

−Val−Val−Leu−Gly−Phe

−Pro−Cys−Asn−Gln−Phe

−Gly−His−Gln−Glu−Asn

−Cys−Gln−Asn−Glu−Glu

−Ile−Leu−Asn−Ser−Leu

−Lys−Tyr−Val−Arg−Pro

−Gly−Gly−Gly−Tyr−Gln

−Pro−Thr−Phe−Thr−Leu

−Val−Gln−Lys−Cys−Glu

−Val−Asn−Gly−Gln−Asn

−Glu−His−Pro−Val−Phe

−Ala−Tyr−Leu−Lys−Asp

−Lys−Leu−Pro−Tyr−Pro

−Tyr−Asp−Asp−Pro−Phe

−Ser−Leu−Met−Thr−Asp

−Pro−Lys−Leu−Ile−Ile

−Trp−Ser−Pro−Val−Arg

−Arg−Ser−Asp−Val−Ala

−Trp−Asn−Phe−Glu−Lys

−Phe−Leu−Ile−Gly−Pro

−Glu−Gly−Glu−Pro−Phe

−Arg−Arg−Tyr−Ser−Arg

−Thr−Phe−Pro−Thr−Ile

−Asn−Ile−Glu−Pro−Asp

−Ile−Lys−Arg−Leu−Leu

−Lys−Val−Ala−Ile    (Ⅰ)

wherein * * * is selenocystein.

In the coding region of this gene, there are homologies for 61.0 % in nucleothides and 66.1 % in amino acids as compared with known h-GSHPx of human liver. (Nucleic Acids Research, Vol. 15, No. 17, pp 7178 (1987)], and hence it refers to a novel gene. Further according to comparative studies of gene expression by Northern blot technique with known two kinds of h-GSHPx gene, h-GSHPx of the present invention is found to be a gene coding a main h-GSHPx in a cell which express major h-GSHPx in liver, and the known h-GSHPx genes was a gene an isomer of the present h-GSHPx which express minor isomer thereof.

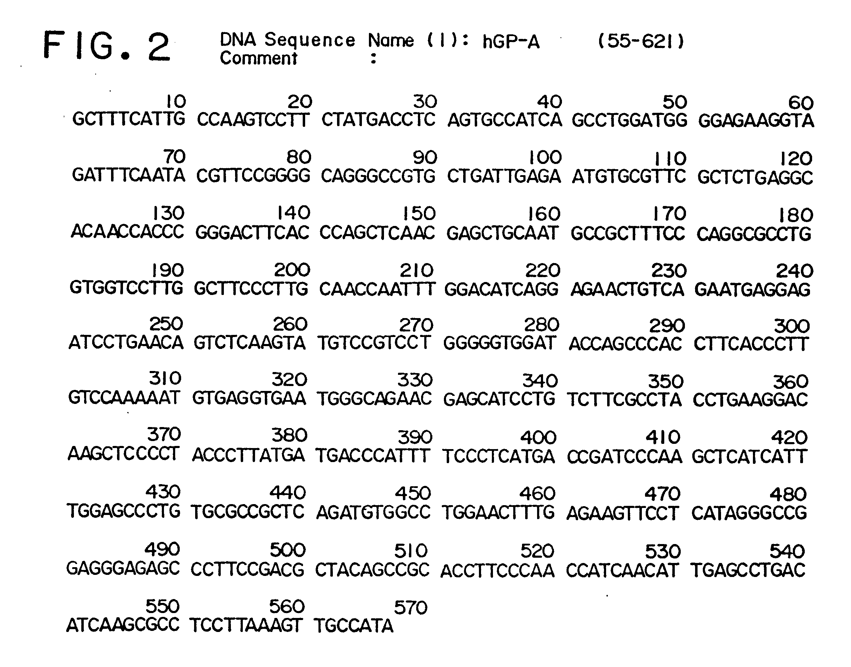

We have also obtained a polypeptide comprising h-GSHPx of the formula [ I ] hereinabove, and found that a base sequence thereof is expressed base No. 1 to No. 567 from 5′-termninal of its sequence in Fig. 2, and that it is a novel gene having an opal-codon (TGA) which codes selenocystein.

Further, we have created the transformant to which tranforming a DNA containing base sequence coding an amino acid sequence of polypeptide comprising h-GSHPx of the present invention by genetic engineering technique. We have also establish a mass production process for a polypeptide comprising a novel h-GSHPx by culturing the thus obtained transformant.

An object of the present invention is to provide a DNA which comprises at least coding an amino acid sequence of a polypeptide comprising of glutathion peroxidase having a structure from N-terminal of the formula [ I ]

Ala - Phe - Ile - Ala - Lys

- Ser - Phe - Tyr - Asp - Leu

- Ser - Ala - Ile - Ser - Leu

- Asp - Gly - Glu - Lys - Val

- Asp - Phe - Asn - Thr - Phe

- Arg - Gly - Arg - Ala - Val

- Leu - Ile - Glu - Asn - Val

- Arg - Ser - Leu - * * * - Gly

- Thr - Thr - Thr - Arg - Asp

- Phe - Thr - Gln - Leu - Asn

- Glu - Leu - Gln - Cys - Arg

- Phe - Pro - Arg - Arg - Leu

- Val - Val - Leu - Gly - Phe

- Pro - Cys - Asn - Gln - Phe

- Gly - His - Gln - Glu - Asn

- Cys - Gln - Asn - Glu - Glu

- Ile - Leu - Asn - Ser - Leu

- Lys - Tyr - Val - Arg - Pro

- Gly - Gly - Gly - Tyr - Gln

- Pro - Thr - Phe - Thr - Leu

- Val - Gln - Lys - Cys - Glu

- Val - Asn - Gly - Gln - Asn

- Glu - His - Pro - Val - Phe

- Ala - Tyr - Leu - Lys - Asp

- L y s - L e u - P r o - T y r - P r o

- T y r - A s p - A s p - P r o - P h e

- S e r - L e u - M e t - T h r - A s p

- P r o - L y s - L e u - I l e - I l e

- T r p - S e r - P r o - V a l - A r g

- A r g - S e r - A s p - V a l - A l a

- T r p - A s n - P h e - G l u - L y s

- P h e - L e u - I l e - G l y - P r o

- G l u - G l y - G l u - P r o - P h e

- A r g - A r g - T y r - S e r - A r g

- T h r - P h e - P r o - T h r - I l e

- A s n - I l e - G l u - P r o - A s p

- I l e - L y s - A r g - L e u - L e u

- L y s - V a l - A l a - I l e   ( I )

wherein * * * is selenocystein.

Another object of the present invention is to provide a transformant which comprises carrying a DNA which has a base sequence coding an amino acid sequence of a polypeptide comprising glutathione peroxidase which is extraneous for the host.

Further object of the present invention is to provide a polypeptide comprising h-GSHPx expressed by the amino acid sequence hereinabove.

More further object of the present invention is to provide a process for production of h-GSHPx which comprises culturing the transformant carrying DNA which has a base sequence of a polypeptide comprising h-GSHPx hereinabove which is extraneous for the host in a medium containing selenium, expressing a genetic information of the said DNA, and isolating a polypeptide comprising h-GSHPx from the cultured medium.

The h-GSHPx gene of the present invention can be prepared by applying commercially available human liver c-DNA library or a library obtained by integrating a DNA fragment using m-RNA prepared from liver into a phage vector. For examples, powdered human liver tissue is homogenized with guanidium solution. A suspension is passed several times through 18 · 1/2 gauge syringe needle to cleave high molecular DNA to the fragment which is layered on 5.7M cesium chloride solution, and centrifuged at 36,000 rpm at 25°C for overnight. The sedimentate is washed with small amount of ethanol and dissolved in water with adding sodium acetate up to 0.3M, then 2.5 volumes of ethanol is added thereto. The re-sedimented total RNA is collected by centrifugation, dissolved in 0.5M NaCℓ solution, heated at 65°C for 5 minutes, thereafter immediately cooled. The solution is charged on a column of oligo(dT)-cellulose and RNA having poly-A tail is eluted. The poly (A) $^+$ RNA is annealed with oligo(dT). The first single strand DNA is synthesized by using reverse transcriptase, and the second single strand DNA is synthesized by using RNase H and DNA polymerase I. EcoRI site in the gene is methylated by EcoRI methylase, and the terminal is subjected to blunt end by T$_4$ DNA polymerase. EcoRI linker DNA is linked to both terminals and the DNA is digested with restriction enzyme EcoRI, then fractionated by electrophoresis, simultaneously removing off the excessively added linker. Fractionated polynucleotide is linked to phage vecotr λgt11. Then c-DNA library consisting of λgt11 vector in which the DNA fragments prepared originally from m-RNA of liver are integrated.

The above c-DNA library is screended with using a probe which is synthesized from several tens to hundreds bases including a codon TGA which codes selenocysteine with reference to the known GSHPx gene. For example, a synthetic oligonucleotide including a codon TGA for selenocysteine, which codes amino acid sequence of No. 31(Ser) to No. 57(Asn) from N-terminal (Cys), in known mouse GSHPx gene is used as a probe for screening.

A host cell, Escherichia coli LE392, infected with λgt11 vector of c-DNA library, is cultured on an agar medium to lyse grown cells. Phage is adsorbed on a nylon membrane filter covered on a surface of the lysate medium. The filter is treated with alkaline to denature DNA, neutralized and fixed the DNA by heating at 80°C for 2 hours. The thus treated filter is kept at 42°C for 60 minutes in a prehybridization solution [e.g. 5 × Denhart solution, 5 × SSC (sodium chloride + sodium citrate), 50 mM sodium phosphate pH 6.5, 0.1 % SDS (sodium dodecyl sulfate), 250 μg/ml exogeneous DNA and 50 % formamide ] . $^{32}$P-labelled DNA probe hereinbefore is added to the solution and hybridized at 42°C for overnight. The treated filter is washed three times in 2 × SSC and 0.1 % SDS at room temperature and successibly, at 50 °C 0.1 × SSC and 0.1 % SDS at 50°C and air-dried. Upon preparing autoradiogram, plasques observed with single are recovered, and re-inoculatedon the agar plate for pure isolation of the plasque to make screening the phage clone including h-GSHPx gene.

The host cells infected with the isolated pure phage are cultured in a liquid medium for overnight and centrifuged to recover the supernatant solution by centrifugation.

20 % polyethylene glycol containing 2.5M NaCl is added thereto, ice-cooled and centrifuged at 15000 rpm for 20 minutes. The precipitate dissolved in SM (0.1M NaCl, 8mM MgSO₄, 50mM Tris-HCl pH 7.5 and 0.02% gelatin solution) and extracted with phenol to extract the DNA. The thus extracted DNA is digested with restriction enzyme EcoRI and purified to prepare the insert including h-GSHPx gene, which is subcloned by ligation into the EcoRI site in a plasmid pUC 118. Thus the plasmid pUC 118-GPA in which the insert including h-GSHPx gene is inserted in EcoRI site in plasmid pUC 118, is prepared. In the subcloning hereinabove, the plasmid pUC 118 can be replaced by a plasmid having a preferable restriction enzyme site, for example a plasmid for the host bacteria belonging to Escherichia coli, such as pBR 322, pBR 325, pACYC 184, pUC 12, pUC 18 or pUC 19, and a plasmid for the host bacteria, Bacillus subtilis, such as pUB 110 or pC 194.

Further in order to confirm an expression for h-GSHPx activity by the plasmid pUC 118-GPA, suitable expression vecotr is assembled. Preferable examples of the expression vecotr are plasmid pINI and pINIII for the host bacteria Escherichia coli such as E. coli DH1, E. coli HB 101, E. coli MV 1304, E. coli W3110 or E. coli C 600 ; plasmid pTUB 218 and pTUB 285 for Bacillus subtilis ; plasmid pAM 82 for Saccharomyces cerevisae ; and virus vector which expresses an extraneous gene by late promoter of SV 40 virus, such as plasmid pSVL (pharmacia Co) or plasmid pSV 2-dhfr (BRL Co) for an animal cells such as primate kidney (COS) cells, Chinese hamster ovary (CHO) cells or CHO-dhfr (dehydrofolate reductase defective) cell-line. These expression vectors can be digested for splitting by restriction enzyme and any sites thereof are added or cut off ligation with the site of DNA fraction including h-GSHPx gene to be inserted.

Expression of h-GSHPx activity, for example in CHO cells can be confirmed as follows. DNA fragments containing h-GSHPx gene is obtained by digesting and splitting the plasmid pUC 118-GPA with restriction enzyme and are inserted into the part of dhfr gene by replacing the said region in the plasmid pSV 2-dhfr. By these procedures, DNA fragments for h-GSHPx gene expression which is prepared by linking with a downstream of an early gene promoter of SV 40, are re-inserted in the EcoRI site of plasmid pSV2-dhfr to obtain the expression vector plasmid pSV2-GPA-dhfr.

The thus prepared expression vector is transferred into the host cells by the known process for example a calcium phosphate method in which the expression vector obtained by the above procedure is transferred into COS cells or CHO cells, a competent cell method and polyethylene glycol method to obtain the transformant.

The transformant, such as CHO cells holding the plasmid pSV2-GPA-dhfr, can be cultured in a medium containing selenium by a conventional cell culture method for animal cells such as CHO cells. For examples, the transformant cells are spread in a culture medium, such as in petridishes, containing the modified Dulbecco-Eeagle MEM medium + 10 % fetal bovine serum + 0.01 ~ 0.05 μM selenious acid, at approximately $10^4$ cells/cm² and cultured at 30 ~ 37 °C for 2 ~ 6 days. Cultured cells are collected and crushed, then the aqueous supernatant, in which h-GSHPx activity is confirmed to express h-GSHPx, is treated by conventional known methods for enzyme isolation and purification to recover h-GSHPx. A solution containing h-GSHPx can be treated by preferable methods such as vacuum concentration, membrane concentration, salting out with ammonium sulfate or sodium sulfate, or fractionation precipitation using water-miscible organic solvent such as methanol, ethanol or acetone. The precipitate containing h-GSHPx is purified if required, by various methods such as dialysis, ion-exchange chromatography, adsorption chromatography or gel-filtration. The thus purified h-GSHPx is stored by lyophilization.

The plasmid pUC 118-GPA containing h-GSHPx producing gene has been holding in Escherichia coli DH1 and was designated as a strain Escherichia coli DH1 pUC 118-GPA, and deposited as FERM BP-1903 under Budapest Treaty in Fermentation Research Institute, Japan.

A gene consisting of 951 base pairs extended from poly-A tail containing h-GSHPx gene shown in Fig. 3A and 3B can be obtained by using the above plasmid. The said gene has an open leading frame including an initiation codon (ATG at position No. 1 ~ 3) to a termination codon (TAG at position No. 571 ~ 573). The base sequence of the above gene is determined by sequencer kit.

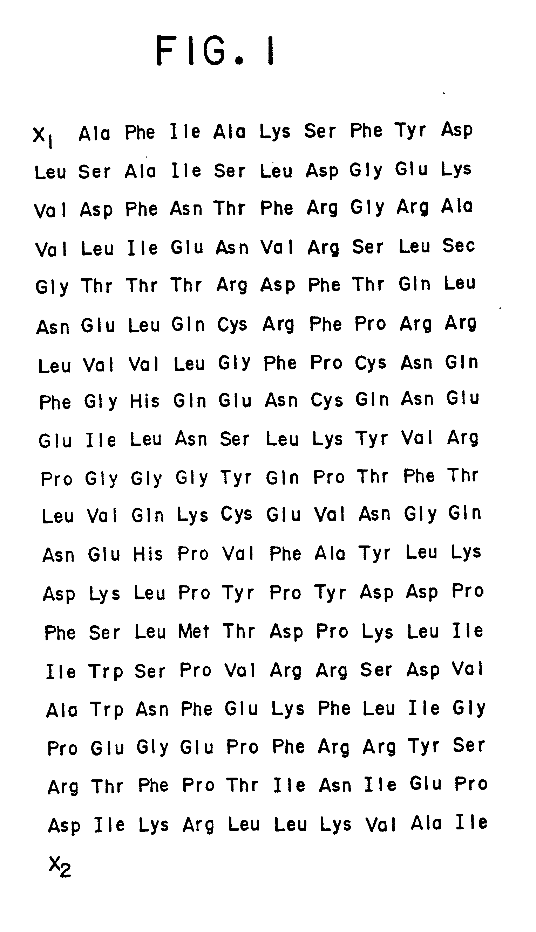

The h-GSHPx is coding at least a polypeptide consisting of 189 amino acids shown in Fig. 1. In Fig. 1, $X_1$ is optionally be a hydrogen, acetyl, methionine or a signal peptide including methionine. The said structure gene shows homology for the knwon h-GSHPx of human hepatocytes (Nucleic Acids Res. Vol. 15, No. 17, pp 7178 (1987)] with 61.0 % in nucleotide and 66.1 % in amino acids, and it is a novel gene. A base sequence for the polypeptide consisting of h-GSHPx in Fig. 1 is illustrated as from base No. 1 to No. 567 from 5'-terminal in Fig. 2, and has opal codon (TGA) coding selenocystein, and also it is a novel gene. An upstream codon from GCT at 5'-terminal in Fig. 2 can be any codon which codes amino acids and it can have more them one codon coding amino acids, at 5'-terminal, and is preferably be ATG or a polydeoxyribonucleic acid corresponding to signal peptide. A downstream codon from ATA at 3'-terminal can be a termination codon or a codon coding a amino acid or peptide. Further when in the 3'-terminal region more than one codon which codes amino acid or peptide is existed, additional termination codon is preferably necessary at 3'-terminal side.

An amino acid sequence of polypeptide of h-GSHPx, which is produced by expressing the novel DNA coding an amino acid sequence of polypeptide consisting of h-GSHPx, can be determined by the base sequence of said DNA. An amino acid sequence at N-terminal of polypetide consisting of h-GSHPx is determined by a liquid phase protein sequencer (Beckman Co) and is confirmed as indentical at N-terminal thereof with an amino acid sequence determind by DNA base sequence. Accordingly the polypeptide consisting od h-GSHPx of the present invention has an amino acid sequence at least illustrated as the formula [ I ] hereinbefore.

A transformant can also be obtained by applying expression vector in preferable host microorganisms instead of CHO cells hereinabove. Examples of microbial transformant is preferably a microorganism belonging to genus Escherichia coli, which is cultured in a medium for expressing h-GSHPx. Cultivation can be performed in a liquid culture medium, and a submerged airation culture is preferable for industrial production.

A conventional medium for culturing microroganisms can preferably be used. For the carbon sources, assimilable carbon sources such as glucose, sucrose, molasses, glycerol, starch hydrolysate or the like can prefereably be used. Assimilable nitrogen sources such as corn steep liquor, Soy been powder, peptone, meat extract, yeast extract, casein hydrolyzate, ammonium sulfate, ammonium chloride or the like can be used. Various inorganic salts such as phosphates, chloride, carbonates, sulfates or nitrate salts of sodium magnesium, calcium, potassium, iron, manganese, zinc or copper can be used. Further seleneous is added at 0.05 ~ 10 μM in a medium.

The culturing temperature can be selected within the range for growth of microbial cells and production of h-GSHPx, and is preferably 20 ~ 42 °C for E. coli. The culturing time can be altered depending on conditions and is terminated when the h-GSHPx production is substantially complete, and is usually 12 ~ 72 hours.

To separate h-GSHPx from the culture, whole cultured mass is used. In case that h-GSHPx is an endoenzyme, a cultured mass is filtered or centrifuged to collect the cells, which are disrupted by treatment with mechanical means or enzymes such as lysozyme. Further if necessary h-GSHPx is solubilized by adding chelating reagent and a surfactant to extract the enzyme.

The enzyme can be purified by the conventional means as hereinbefore illustarted.

An activity of h-GSHPx is determined by the followings. A solution of h-GSHPx 10μℓ is mixed with a solution (0.98 mℓ) containing 0.1M Tris-HCℓ buffer (pH 8.0), 0.2 mM reduced NADP, 0.5 mM EDTA, 2 mM glutathione and 1 unit of glutathione reductase, added butanol peroxide (Bu 00H), and the decreasing absorbance of reduced NADP at 340 nm by oxidation thereof is spectrophotometrically measured at 37°C. By this means expression and production of h-GSHPx can be confirmed.

Abbreviations of amino acid, peptide, nucleic acid and others are illustrated according to commonly used notation. All the amino acid shows L-form.

Following examples illustrate the present invention but are not construed as limiting.

Example 1

Human liver 10g was powdered in a liquid nitrogen, homogenized in 6M guanidine isothiocyanate (40mℓ) and passed through 18.5 G injection needle to reduce viscosity with splitting high molecular DNA. A homogenate was layered on a 1/3 volume solution of 5.7M cesium chloride and 0.1M EDTA (pH 7.5), and centrifuged at 35,000 rpm at 25°C for 18 hours. A precipitate was washed with small amount of ethanol to remove excess cesium chloride. The precipitate dissolved in water (1 mℓ) was centrifuged after adding 1/10 volume of 3M sodium acetate and 2.5 volumes of ethanol, collected the precipitate and dried in vacuo to obtain crude RNA (5 mg).

The precipitate dissolved in water (1.5 mℓ), heated at 65 °C for 5 minutes was immediately cooled and added a solution (1.5 mℓ) of 40 mM Tris-HCℓ (pH 7.6), 1.0 M NaCℓ, 2 mM EDTA and 0.2 % SDS therein. The solution was adsorbed to a column of oligo (dT) cellulose (Pharmacia Co) (75 mg) equilibrated with 20 mM Tris-HCℓ (pH 7.6), 0.5M NaCℓ, 1 mM EDTA and 0.1 % SDS, and washed with the same solution (10 mℓ). RNA except poly (A)+ RNA was eluted with a solution (5 mℓ) of 20 mM Tris-HCℓ (pH 7.6), 0.1M NaCℓ, 1 mM EDTA and 0.1 % SDS.

Poly (A)+ RNA was eluted with solution of 10 mM Tris-HCℓ (pH 7.5), 1 mM EDTA and 0.05 % SDS, and the first eluted fraction (1 mℓ) as collected. A 1/10 volume of 3M sodium acetate and 2.5 volumes of ethanol were added thereto, allowed to stand at -20 °C for overnight, centrifuged at 15,000 rpm for 30 minutes to collect the precipitate and lyophilized. Poly (A)+ RNA (50 μg) was obtained.

Example 2

Poly (A)+ RNA obtained in Example 1 was dissolved to 1 μg/mℓ in water. The solution (5 μℓ) transferred into a micro-tube was heated at 65 °C for 5 minutes and immediately cooled. 50 mM Tri-HCℓ (pH 8.3), 10 mM MgCℓ2, 140 mM KCℓ, 10 mM dithiothreitol and 2 mM d NTPs (equivalent mixture of dATP, dGTP, dCTP and dTTP), oligo(dT) (5 μg) (Pharmacia Co) and reverse transcriptase (1.5 unit) (Takara Shuzo Co) were added thereto. Total volume of the mixture was set up to 20μℓ and incubated at 42°C for 1 hour. 80 mM Tris-HCℓ (pH 7.5), 200 mM KCℓ, 10 mM MgCℓ2, 25 μg/mℓ BSA and RNase H (Takara shuzo Co) (60 units) and DNA polymerase I (Boehringer Mannheim Co) (5 units) were added to the incubated mixuture above and set up total volume to 150 μℓ. The mixuture was reacted at 120 °C for 1 hour and later at 22°C for 1 hour. 0.25M EDTA (20µℓ) and 10% SDS (10 µℓ) were added to stop the reaction, and an equal amount of phenol-chloroform was added, then centrifuged at 10,000 rpm for 5 minutes. Aqueous layer was collected and an equal amount of 4M ammonium acetate and twice volume of ethanol were added thereto. The mixture was centrifuged at 15,000 rpm for 15 minutes, collected the precipitated and dried in vacuo. 100 mM Tri-HCℓ (pH 8.0), 10 mM EDTA, 80 µM S-adenocylmethionine, 100µg/mℓ BSA and 2 unit EcoRI methylase (Promegabiotech Co) were added thereto and set up to 10 µℓ, then the mixture was incubated at 37 °C for 1 hours. Water (40 µℓ) was added to the incubated mixture, treated with an equal amount of phenol-chloroform and an equal amount of 4M ammonium acetate and twice volume of ethanol were added to the aqueous layer which was separated by centrifugation, thereafter allowe dto stand at -70°C for 15 minutes. 67 mM Tris-HCℓ (pH 8. 8), 6.7 mM MgCℓ$_2$, 16.6 mM ammonium sulfate, 10 mM 2-mercaptoethanol, 6.7 µM EDTA, 0.167 % BSA, each 750 µM of dATP, dGTP, dCTP, dTTP, and T4 DNA polymerase (Takara Shuzo Co) (4 units) were added to the precipitate obtained by centrifugation at 15,000 rpm for 15 minutes. The mixture was set up total volume to 12µℓ and incubated at 37°C for 1 hour. Reaction mixture was treated with an equal amount of phenol-chloroform. Ethanol precipitate was collected by centrifugation and dried in vacuo. EcoRI linker (1µg) mixed with 50 mM Tris-HCℓ (pH 7.6), 10 mM MgCℓ$_2$, 10 mM dithiothreitol, 0.1 mM spermidine, 0.1 mM EDTA, 1 mM ATP and T4 polynucleotide kinase (3 units) (Takara Shuzo Co) was set up a volume to 10µℓ and the mixture was incubated at 37 °C for 30 minutes. Total volume of this mixture was added to the sample which was treated previously with T4 DNA polymerase, added T4 ligase(60 units) (Pharmacia Co), then reacted at 14°C for overnight.

100 mM NaCℓ, 50mM Tris- HCℓ (pH 7.5), 10mM MgCℓ$_2$, 7mM 2-mercaptoethanol, 100µg/mℓ BSA and EcoRI (250 units) were added to the reaction mixture, and set up total volume to 40µℓ, then incubated at 37°C for 2 hours. The reaction mixture was fractionated by 1% low melting point agarose gel and a gel containing 600-2000 base DNA was collected. After melting the gel at 65 °C for 10 minutes, an equal amount of phenol was added thereto, subject to ice-cooling for 10 minutes , then centrifuged at 4 °C for 10 minutes at 15,000 rpm. An equal amont of phenol was added to the aqueous layer, and the above operations were repeated, thereafter phenol treatments were again repeated. 1/10 volume 3M sodium acetate and 2.5 volume of ethanol were added to the aqueous layer which was previously treated with chloroform, and the mixture was allowed to stand at -70 °C, then centrifuged at 15,000 rpm for 15 minutes. The precipitate washed twice with 75% ethanol was dried in vacuo. A λ phage vector λgt11 arm (Stratagene Co) (1µg) was added thereto and reacted with using ligation kit (Takara Shuzo Co) at 26 °C for 10 minutes. Thereafter a sample was reacted with using in vitro packaging kit (Stratagene Co). The thus obtained λ phage was infected in Escherichia coli LE 392 which was counted in total 8.0× 10$^5$ pfu (plaque forming units).

Example 3

The prepared human liver c-DNA library 8.0× 10$^5$ pfu was spread with an indicator bacterium E. coli LE 392 (purchased form Stratagene Co) on a medium of 1.5 % LB agar medium (bactotrypton 10g, bactoyeast extract 5g and NaCℓ 10g in 1ℓ] , and GSHPx clone was selected by plaque hybridization. Lytic plsque on the LB plate was transferred to a nylon membrane, and it was then treated with 0.5M NaOH and 1.5M NaCℓ for 5 minutes and 3M sodium acetate (pH 5.5) for 5 minutes, and dried at 80°C for 2 hours in vacuo. The membrane was put in the vinyl package, and treated with (10 mℓ) 5 × SSC [1 × (150 mM NaCℓ and 15 mM sodium citrate)] , 5 × Denhart solution (0.002 % Ficoll, 0.02 % polyvinylphyrrolidone and 0.022 % BSA), 50 mM sodium phosphate (pH 6.5), 0.1 % SDS, 250µg/mℓ salmon sperm DNA and 50 % formamid at 42°C for 60 minutes. After removing the liquid mixture, synthetic oligonucleotide, in which 5′-terminal was labelled with $^{32}$P (10$^8$ cpm/µg), 5′GTTCATCTCGGTGTAGTCCCGGATCGTGGTGCCTCAGAGAGACGCGACATTCT- CAATGAGCAGCACCTTGCCCCGCAGGGA3′ which is a base sequence coding an amino acid sequence, Ser, Leu, Arg, Gly, Lys,

Val, Leu, Leu, Ile, Glu, Asn, Val, Ala,

Ser, Leu, Sec, Gly, Thr, Thr, Ile, Arg,

Asp, Tyr, Thr, Glu, Met, Asn

containing a region coding selenocystein of mouse GSHPx, (20 ng) was added thereto and hybridized at 42°C for overnight. The filter was washed three times with 2 × SSC and 0.1 % SDS at room temperature and at 50 °C for 10 minutes, then washed with 0.1 × SSC and 0.1 % SDS at 50°C for 10 minutes, dried with aeration and subjected to make autoradiogram. A part of medium which showing positive signalwas collected, diluted with SM [0.1M NaCℓ, 8mM MgSO$_4$ and 50 mM Tris-HCℓ (pH 7.5) and 0.01 % gelatine ] , then again spread on the LB plate. Plaque was selectively purified with the same probe hereinabove for screening, and thus 10 clones were obtained.

Examples 4

A recombinant λ phage obtained in Example 3 was infected in the host cell LE 392 and cultured with shaking in LB medium (10 mℓ) for overnight. Cultured medium was centrifuged at 8,000 rpm for 10 minutes. DNase I (Takara Shuzo Co) (60 units) and RNase A (Sigma Co) (100µg) were added to the supernatant solution and incubated at 37 °C for 30 minutes. An equal amount of 20 % polyethylene glycol and 2.5M NaCℓ were added thereto, and incubated onice for an hour and centrifuged at 15,000 rpm for 20 minutes. The precipitate was suspended in 0.5mℓ SM, treated with an equal amount of phenol and centrifuged. The aqueous layer was treated with chloroform-phenol, added 1/10 volume of sodium acetate and 2 volumes of ethanol, and allowed to stand at -70°C for 15 minutes. A precipitate collected centrifugation at 15,000 rpm for 15 minutes was washed twice with 75 % ethanol and dried in vacuo. The precipitate was dissolved in water (50µℓ) containing 5µg/mℓ RNase A. Complete digestion of the inserted fragment in a solution (10 µℓ) with EcoRI showed to obtain h-GSHPx clone of the maximum length approximately 1kb. Inserted fragment of approximately 1kb was recovered from the low melting point agarose gel.

Separately a vector pUC 118 was completely digested with EcoRI, added bacterial alkalinephosphatase (0.5 unit) (Toyobo Co) in 50 mM Tris-HCℓ (pH 8.0), and incubated at 65°C for 1 hour. A reaction mixture was treated twice with chloroform-phenol, added 1/10 volume of 3M sodium acetate and 2 volumes of ethanol to the aqueous layer, and centrifuged to collect the vector. The inserted fragment recovered from the gel and the vector pUC 118 digested with EcoRI were ligated by using ligation kit (Takara Shuzo Co).

Logarithmic growth E. coli MV 1304 (purchased form Takara Shuzo Co) cultured in Ψ medium (bactotriptone 20g, bactoyeast extract 5g and MgSO₄ 14g, in 1ℓ, pH 7.6) (100 mℓ) was collected, suspended in an ice-cooled solution (40 mℓ, pH 5.8)) of 30 mM pottasium acetate, 100 mM RbCℓ, 10 mM CaCℓ₂, 50 mM MnCℓ₂ and 15 % glycerol, and allowed to stand at 0 °C for 5 minutes. Bacterial cells were collected by centrifugation, suspended in a solution (4mℓ, pH 6.5) of 10 mM MOPS buffer (Dotai Co), 75 mM CaCℓ₂, 10 mM RbCℓ and 15 % glycerol, and allowed to stand at 0 °C for 15 minutes to prepare competent cells. The Ligated DNA solution (20µℓ) hereinabove was added to the E. coli suspension (200µℓ), and allowed to stand at 0°C for 30 minutes. The suspension was treated at 42°C for 90 seconds, added LB medium (800µℓ) thereto and incubated at 37 °C for 60 minutes. The incubated suspension (300µℓ) was spread on LB agar plate containing ampicillin 50µg/mℓ, 0.02% X-gel (5-bromo-4-chloro-3-indolyl-β-galactoside) and 50µM IPTG (isopropyl-β-D-thiogalactopyranoside), then cultured for overnight to obtain the transformant. Transformed single white colony was cultured in LB medium (2mℓ) for overnight and was collected by centrifugation. A solution (0.6 mℓ) of 50 mM Tris-HCℓ (pH 8.0), 50 mM EDTA (pH 8.0) and 15 % sucrose, containing 1 mg/ mℓ lysozyme (Sigma Co) was added to the collected cells and incubated at 37°C for 15 minutes. 10 % SDS (12µℓ) was mixed therewith, added 5M potassium acetate therein and was allowed to stand at 0 °C for 30 minutes. The reaction mixture was centrifuged at 15,000 rpm for 10 minutes. The upper layer was treated with an equal amount of chloroform-phenol, then the aqueous layer was treated twice with ether, added 2 volumes of ethanol and allowed to stand at -70°C for 15 minutes. Precipitate collected by centrifugation at 15,000 rpm for 15 minutes was washed twice with 75 % ethanol and dried in vacuo. The precipitate dissolved in a water containing 5µg/mℓ RNase, was digested with EcoRI to select the clone containing insertion fragment.

Example 5

Single colony containing the clone hereinabove was cultured at 37°C for overnight in LB medium (6 mℓ), and the cultured medium (5 mℓ) was inoculated in LB medium (500 mℓ) and cultured at 37°C. Chloramphenicol (20µℓ/mℓ) was added to the logarithmic growth cells, further cultured for overnight. Lysozyme dissolved (10 mg/mℓ) (1.5 mℓ) in a solution (15 mℓ) of 8 % sucrose, 10 % Triton-X, 25 mM EDTA, 50 mM Tris-HCℓ (pH 8.0) and 0.25 M, Tris-HCℓ (pH 8.0), was added to the cells cpllected by centrifugation at 6,000 rpm for 10 minutes and heated to lysate. Supernatant solution obtained by centrifugation at 14,000 rpm for 30 minutes was treated with an equal amount of chloroform-phenol, and 1/10 volume of 3M sodium acetate and 2 volumes of ethanol were added to the aqueous layer, then allowed to stand at -70°C for 15 minutes. A precipitate collected by centrifugation at 3000 rpm for 15 minutes was dissolved in Tris-HCℓ (pH 7.4) (21 mℓ), added Cesium chloride (20g) and 10 mg/mℓ ethidium bromide (1 mℓ) thereto and centrifuged at 50,000 rpm at 4°C for overnight. Closed circular plasmid DNA was fractionated and treated three times with an equal amount of butanol to remove ethidium bromide. The DNA sample was gel-filtrated through CL4B Sepharoe gel (Pharmacia Co) washed with Tris-HCℓ (pH 7.4) containing 0.2M NaCℓ to remove impurities, added 2 volumes of ethanol thererto and allowed to stand at -70°C for 15 minutes. Precipitated collected by centrifugation at 3000 rpm for 30 minutes was washed twice with 75 % ethanol, dried and adjusted to concentration of 1 µg/ µℓ.

Single strand DNA of each pair positive and negative strand chain for whole domain was prepared and the base sequence was determined by dideoxy method (Science, 214: 1205-1210 (1981)] . (Fig. 3).

Further this plasmid (0.5 µg) was transformed into E. coli DH1 competent cells (provided by National Institute of Genetics, Japan), which was treated as same as of the above E. coli MV 1304, and was inoculated in LB agar medium containing ampicillin 50µg/mℓ then cultured for overnight to obtain the transformant. This plasmid containing human GSHPx was designated as Escherichia coli DH1 pUC 118-GPA.

Example 6

EcoRI (15 units) was added to pUC 118-GPA DNA (10 µg) in H-buffer (Maniatis, et al. Molecular cloning, 104 : Cold Spring Harbor (1982)] , set up total volume to 20 µℓ and subjected to cleave at 37 °C for 2 hours.

DNA fragment of approximately 990 base pair was extracted by 1 % low melting point agarose gel electrophoresis, purified and dried.

Separately, Bluescript KS (Stratgene Co) DNA (1 μg) in H-buffer hereinbefore was mixed with EcoRI to set up total volume of 20μℓ and was subjected to cleave at 37°C for 2 hours. 1M Tris-HCℓ (pH 8.0) (5 μℓ) and distilled water (75 μℓ) was added to the reaction mixture, and the mixture was treated with bacterial alkaline phophatase (1 unit) at 65°C for 1 hour. (hereinafter this treatment with alkaline phosphatase is designated as BAP-treatment)

DNA was recovred, after twice treatment with an equal amount of chloroform-phenol, by ethanol precipitation, and dried in vacuo.

The 990 base pair DNA fragment and BAP treated vector were dissolved in water (17 μℓ) and 10 times concentration of ligation buffer (2 μℓ) and T4 DNA ligase (Takara Shuzo Co., 350 U/ μℓ) (1 μℓ) were added thereto, then allowed to stand at 14°C for overnight.

DNA solution reacted hereinabove was added to the competent E. coli DH-1 (200μℓ), allowed to stand at 0°C for 30 minutes, further at 42°C for 90 seconds, added L-medium (800 μℓ) and incubated at 37 °C for 60 minutes. The incubated (100μℓ) was spread on L-agar medium containg ampicillin 50 μg/mℓ, incubated at 37 °C for overnight to obtain the transformant. The thus obtained clone was designated as Bluescript KS-GPA.

The Bluscript KS-GPA DNA (10μg) was added to HindIII (15 units) and BamHI (15 units) and total volume was to up 20μℓ with H-buffer, and subjected to cleave at 37 °C for 2 hour. DNA fragment approximately 1,000 base pairs was extracted from 1 % low melting point agarose electrophoresis, purified and dried.

Separatory therefrom, HindIII (2 units) and BgℓII (2 units) were added to pSV2-dhfr DNA (BRL Co) (1 μg) and total volume was set up to 20 μℓ with adding H-buffer, then subject to cleave at 37°C for 2 hours, thereater the mixture was subjected to BAP- treatment to obtain the vector.

The thus obtained vector and approximately 1,000 base pairs DNA fragment were taken to ligation reaction and transformation to prepare the transformant pSV2-GPA. The pSV2-GPA DNA (10μg) was mixed with EcoRI (15 units) up to 20μℓ with adding H-buffer and reacted to cleave at 37 °C for 2 hours. DNA fragment of approximately 2,970 base pairs was extracted from 1 % low melting point agarose gel, and dried.

Separately therefrom, EcoRI (2 units) was added to pSV2-dhfr DNA (1μg) in H-buffer with which total volume thereof was set up 20 μℓ and reacted to cleave at 37 °C for 2 hours. Thus a vector was obtained by BAP-treatment.

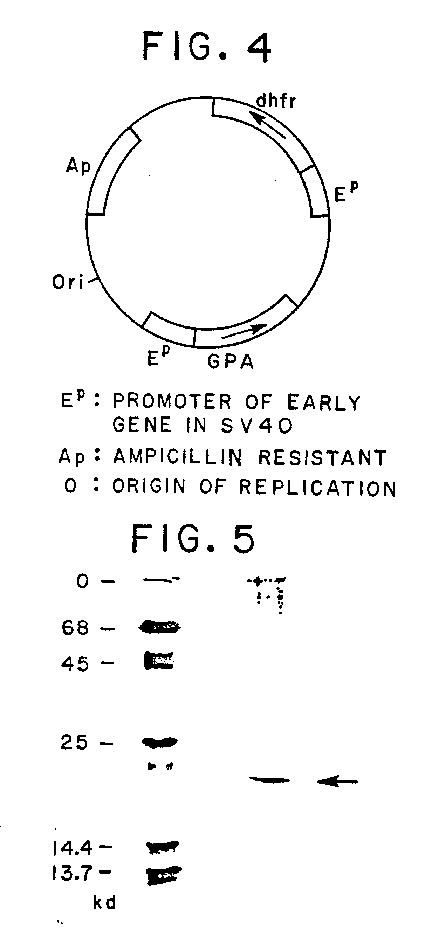

The said vector and the DNA fragment of approximately 2,970 base pairs were subjected to ligation reaction and transformation. The thus obtained transformant was designated as pSV2-GPA-dhfr. (Fig. 4)

Example 7

The solution B consisting of an aqueous solution (2.2 mℓ) of pSV2-GPA-dhfr DNA (100 μg) and 2M CaCℓ₂ (0.3 mℓ) was gradually added dropwise with aeration into the solution A consisting of 2 × HBS (HEPES 10g/ℓ, and NaCℓ 6g/ℓ, pH 7.10) (2.5 mℓ) and 100 × PO₄ (70 mM NaH₂PO₄ + 70 mM Na₂HPO₄) (0.05 mℓ) to precipitate DNA calcium phosphate. The said solution of precipitate (1mℓ) was added to CHO-dhfr-cells grown in HamF-12 medium (Flow Co) containing fetal bovine serum 10 % (10 mℓ) then allowed to stand at 37°C for overnight at 5 % CO₂ amosphare. A medium was changed with another same medium (10 mℓ) and incubated further for 24 hours. Finally the medium was changed D-MEM medium (Gibco Co) cintaining dialysed fetal bovine serum 10 % for selection medium, which was changed once within each four days, and incubated for one month to obtain transformant cells strain CHO-GPA.

The thus obtained transformant cells strain CHO-GPA was incubated in D-MEM medium containing fetal bovine serum 10 % which contains methotraxate (Sigma Co) 1 mM, 10 mM, and 100 mM, respectively, then medium was changed once within each four days and incubated for one month to obtain the transformant CHO-GPA 100 which can grown at methotraxate 100 mM.

Example 8

The CHO-GPA 100 strain was incubated in D-MEM medium containing fetal bovine serum 10 % and 0.02 μM selenious acid and 100 mM methotrexate using plastic petridish, diameter 10cm, 50 plates for 3 days. Incubated cells, approximately 10⁸ cells were suspended 10 mM phosphate buffer (pH 7.0) (100mℓ) containing 0.7 mM 2-mercaptoethanol (100mℓ) and ultra sonicated. Sonicate was centrifuged at 10,000 rpm for 15 minutes. Ammonium sulfate was added to the supernatant enzyme solution, and the precipitate with 25 % ~ 50 % ammonium sulfate saturation was collected by centrifugation at 15,000 rpm for 30 minutes. The precipitate was again dissolved in 10 mM phosphate buffer (pH 7.2) (10 mℓ) containing 0.7 mM mercaptoethanol and dialysed three times against 50 volumes of the same buffer solution. Inner dialysate was adsorbed to a column (2.5 × 20 cm) of DEAE-cellulose DE 52 (Whatman Co) and subjected to gradient elution with a linear gradient of 0 ~ 100 mM NaCℓ in the same buffer solution. Active fractions eluted with NaCℓ concentration 45 ~ 75 mM were collected and concentrated, and subjectd to molecular-sieving with Sephadex G-200 (Pharmacia Co) to obtain enzymatic active fraction.

The said active fraction was adsorbed to a column (1.5 × 10 cm) of DEAE-Sephadex (Pharmacia Co) and eluted with 5 mM phosphate buffer (pH 7.0) containing 0.7 mM 2-mercaptoethanol with linear gradient of 0 ~ 100 mM NaCℓ concentration. Enzyme active fractions eluted within NaCℓ concentration 10 ~ 20 mM were dialysed three times against 50 volumes of the same buffer solution, then inner solution was concentrated to obtain h-GSHPx specimen. Total protein of the said sample was determined by Lowry method [J. Biol.

Chem. 193 : 263-275 (1951)] to obtain 110 μg GSHPx. A solution of the said sample (1μg) and standard protein for referencce, bovine serum albumin (68 kDa), egg albumin (45 kDa), chymotrypsinogen (24 kDa), lysozyme (14.4 kDa) and RNAase A (13.7 kDa), each 5 μg, were treated with 0.1 % SDS and 0.5M 2-mercaptoethanol at 95 °C for 2 minutes. These proteins were subjected to SDS-polyacrylamide electrophoresis according to Laemmli method [Nature, 227 : 680 - 685 (1970)] . The obtained h-GSHPx showed single band of molecular weight approximately 21 kDa. (Fig. 5)

The h-GSHPx (0.1μg) was mixed with a solution (0.98 mℓ) consisting of 0.1M Tris-HCℓ (pH 8.0), 0.2 mM reduced NADP, 0.5 mM EDTA, 2mM glutathion and glutathion reductase (1 unit) (Sigma Co), added t-butyl hydroperoxide (10 μℓ), final concentration 70 μM) and incubated at 37°C.

Decrease in an absorption at 340 nm of reduced NADP by oxidation was measured. An activity thereof was measured as 0.1 unit, on the condition that an activity converting 1μ mole of glutathione to oxdized form at 37 °C per one minutes, is defined as one unit.

An amino acid sequencce of the sample (10 μg) of h-GSHPx of the present invention from N-terminal amino acid was determined in 0.01M SDS solution by 890ME sequencer (Beckmann Co).

The sequence was determined as follows.

A l a — P h e — I l e — A l a — L y s — S e r —

P h e — T y r — A s p — L e u — S e r — A l a —

I l e — S e r — L e u — A s p — G l y — G l u —

L y s — V a l .

The above amino acid sequence was identical with the amino acid sequence which being coded by the plasmid pUC 118-GPA of the present invention.

[Effect of the invention]

Glutathione peroxidase can be effectively produced by using DNA and transformant of the present invention. Also an amino acid sequence of a novel glutathione peroxidase from human origin was elucidated according to the present invention, and further it was isolated in pure form. Novel glutathione peroxidase gene was also clearly elucidated.

BRIEF EXPLANATION OF THE DRAWINGS

    Fig. 1 : Amino acid sequence of glutathione peroxidase of the present invention ;
    Fig. 2 : Base sequence of glutathione peroxidase gene of the present invention ;
    Fig. 3A and 3B : Polydeoxyribonucleic acid and coding region of amino acids including glutathione peroxidase gene ;
    Fig. 4 : Structural construction of plasmid pSV2-GPA-dhfr ; and
    Fig. 5 : Electrophoresis pattern expressing glutathione peroxidase of the present invention.

**Claims**

1. A DNA sequence which codes for a polypeptide comprising a glutathione peroxidase, the glutathione peroxidase having an amino acid sequence comprising, from the N-terminal:

Ala – Phe – Ile – Ala – Lys

– Ser – Phe – Tyr – Asp – Leu

– Ser – Ala – Ile – Ser – Leu

– Asp – Gly – Glu – Lys – Val

– Asp – Phe – Asn – Thr – Phe

– Arg – Gly – Arg – Ala – Val

– Leu – Ile – Glu – Asn – Val

– Arg – Ser – Leu – *** – Gly

– Thr – Thr – Thr – Arg – Asp

– Phe – Thr – Gln – Leu – Asn

– Glu – Leu – Gln – Cys – Arg

– Phe – Pro – Arg – Arg – Leu –

- Val - Val - Leu - Gly - Phe

- Pro - Cys - Asn - Gln - Phe

- Gly - His - Gln - Glu - Asn

- Cys - Gln - Asn - Glu - Glu

- Ile - Leu - Asn - Ser - Leu

- Lys - Tyr - Val - Arg - Pro

- Gly - Gly - Gly - Tyr - Gln

- Pro - Thr - Phe - Thr - Leu

- Val - Gln - Lys - Cys - Glu

- Val - Asn - Gly - Gln - Asn

- Glu - His - Pro - Val - Phe

- Ala - Tyr - Leu - Lys - Asp

- Lys - Leu - Pro - Tyr - Pro

- Tyr - Asp - Asp - Pro - Phe

- Ser - Leu - Met - Thr - Asp

- Pro - Lys - Leu - Ile - Ile

- Trp - Ser - Pro - Val - Arg

- Arg - Ser - Asp - Val - Ala

- Trp - Asn - Phe - Glu - Lys

- Phe - Leu - Ile - Gly - Pro

- Glu - Gly - Glu - Pro - Phe

- Arg - Arg - Tyr - Ser - Arg

- Thr - Phe - Pro - Thr - Ile

- Asn - Ile - Glu - Pro - Asp

- Ile - Lys - Arg - Leu - Leu

- Lys - Val - Ala - Ile

wherein * * * is selenocystein.

2. A DNA sequence according to claim 1 which comprises the bases No. 1 to No. 567 of Figure 2.

3. A vector which incorporates the DNA sequence of claims 1 or 2 and which is capable, in a transformed host, of expressing the said DNA sequence.

4. A vector according to claim 3, which is a plasmid.

5. A host transformed with a DNA sequence of claims 1 or 2, or with a vector of claims 3 or 4.

6. A transformed host according to claim 5, wherein the host is a bacterial or mammalian cell line.

7. A transformed host according to claim 6, which is E.coli DH1.

8. A transformed host according to claim 6, which is a CHO cell line.

9. A polypeptide comprising a glutathione peroxidase, the glutathione peroxidase having the amino acid sequence defined in claim 1.

10. A process for the production of a polypeptide comprising a glutathione peroxidase as claimed in claim 9, comprising culturing a transformed cell line according to any of claims 5 to 8 in a medium containing selenium, thereby expressing the said glutathione peroxidase sequence, and isolating the expressed product.

# FIG. 1

X₁  Ala  Phe  Ile  Ala  Lys  Ser  Phe  Tyr  Asp

Leu  Ser  Ala  Ile  Ser  Leu  Asp  Gly  Glu  Lys

Val  Asp  Phe  Asn  Thr  Phe  Arg  Gly  Arg  Ala

Val  Leu  Ile  Glu  Asn  Val  Arg  Ser  Leu  Sec

Gly  Thr  Thr  Thr  Arg  Asp  Phe  Thr  Gln  Leu

Asn  Glu  Leu  Gln  Cys  Arg  Phe  Pro  Arg  Arg

Leu  Val  Val  Leu  Gly  Phe  Pro  Cys  Asn  Gln

Phe  Gly  His  Gln  Glu  Asn  Cys  Gln  Asn  Glu

Glu  Ile  Leu  Asn  Ser  Leu  Lys  Tyr  Val  Arg

Pro  Gly  Gly  Gly  Tyr  Gln  Pro  Thr  Phe  Thr

Leu  Val  Gln  Lys  Cys  Glu  Val  Asn  Gly  Gln

Asn  Glu  His  Pro  Val  Phe  Ala  Tyr  Leu  Lys

Asp  Lys  Leu  Pro  Tyr  Pro  Tyr  Asp  Asp  Pro

Phe  Ser  Leu  Met  Thr  Asp  Pro  Lys  Leu  Ile

Ile  Trp  Ser  Pro  Val  Arg  Arg  Ser  Asp  Val

Ala  Trp  Asn  Phe  Glu  Lys  Phe  Leu  Ile  Gly

Pro  Glu  Gly  Glu  Pro  Phe  Arg  Arg  Tyr  Ser

Arg  Thr  Phe  Pro  Thr  Ile  Asn  Ile  Glu  Pro

Asp  Ile  Lys  Arg  Leu  Leu  Lys  Val  Ala  Ile

X₂

# FIG. 2

DNA Sequence Name ( I ): hGP-A     (55-621)
Comment         :

```
            10         20         30         40         50         60
 GCTTTCATTG CCAAGTCCTT CTATGACCTC AGTGCCATCA GCCTGGATGG GGAGAAGGTA

            70         80         90        100        110        120
 GATTTCAATA CGTTCCGGGG CAGGGCCGTG CTGATTGAGA ATGTGCGTTC GCTCTGAGGC

           130        140        150        160        170        180
 ACAACCACCC GGGACTTCAC CCAGCTCAAC GAGCTGCAAT GCCGCTTTCC CAGGCGCCTG

           190        200        210        220        230        240
 GTGGTCCTTG GCTTCCCTTG CAACCAATTT GGACATCAGG AGAACTGTCA GAATGAGGAG

           250        260        270        280        290        300
 ATCCTGAACA GTCTCAAGTA TGTCCGTCCT GGGGGTGGAT ACCAGCCCAC CTTCACCCTT

           310        320        330        340        350        360
 GTCCAAAAAT GTGAGGTGAA TGGGCAGAAC GAGCATCCTG TCTTCGCCTA CCTGAAGGAC

           370        380        390        400        410        420
 AAGCTCCCCT ACCCTTATGA TGACCCATTT TCCCTCATGA CCGATCCCAA GCTCATCATT

           430        440        450        460        470        480
 TGGAGCCCTG TGCGCCGCTC AGATGTGGCC TGGAACTTTG AGAAGTTCCT CATAGGGCCG

           490        500        510        520        530        540
 GAGGGAGAGC CCTTCCGACG CTACAGCCGC ACCTTCCCAA CCATCAACAT TGAGCCTGAC

           550        560        570
 ATCAAGCGCC TCCTTAAAGT TGCCATA
```

EP 0 347 224 A2

# FIG. 3A

EP 0 347 224 A2

```
                                 CGCTCATAAGTGGGCTCAGGCCTCTCTGCGGGGCTCACTCTGCGCTTCACC
        10        20        30        40        50        60
ATGGCTTTCATTGCCAAGTCCTTCTATGACCTCAGTGCCATCAGCCTGGATGGGGAGAAG
MetAlaPheIleAlaLysSerPheTyrAspLeuSerAlaIleSerLeuAspGlyGluLys

        70        80        90       100       110       120
GTAGATTTCAATACGTTCCGGGGCAGGGCCGTGCTGATTGAGAATGTGCGTTCGCTCTGA
ValAspPheAsnThrPheArgGlyArgAlaValLeuIleGluAsnValArgSerLeu***

       130       140       150       160       170       180
GGCACAACCACCCGGGACTTCACCCAGCTCAACGAGCTGCAATGCCGCTTTCCCAGGCGC
GlyThrThrThrArgAspPheThrGlnLeuAsnGluLeuGlnCysArgPheProArgArg

       190       200       210       220       230       240
CTGGTGGTCCTTGGCTTCCCTTGCAACCAATTTGGACATCAGGAGAACTGTCAGAATGAG
LeuValValLeuGlyPheProCysAsnGlnPheGlyHisGlnGluAsnCysGlnAsnGlu

       250       260       270       280       290       300
GAGATCCTGAACAGTCTCAAGTATGTCCGTCCTGGGGGTGGATACCAGCCCACCTTCACC
GluIleLeuAsnSerLeuLysTyrValArgProGlyGlyGlyTyrGlnProThrPheThr

       310       320       330       340       350       360
CTTGTCCAAAAATGTGAGGTGAATGGGCAGAACGAGCATCCTGTCTTCGCCTACCTGAAG
LeuValGlnLysCysGluValAsnGlyGlnAsnGluHisProValPheAlaTyrLeuLys

       370       380       390       400       410       420
GACAAGCTCCCCTACCCTTATGATGACCCATTTTCCCTCATGACCGATCCCAAGCTCATC
AspLysLeuProTyrProTyrAspAspProPheSerLeuMetThrAspProLysLeuIle

       430       440       450       460       470       480
ATTTGGAGCCCTGTGCGCCGCTCAGATGTGGCCTGGAACTTTGAGAAGTTCCTCATAGGG
IleTrpSerProValArgArgSerAspValAlaTrpAsnPheGluLysPheLeuIleGly
```

# FIG. 3B

```
        490        500        510        520        530        540
CCGGAGGGAGAGCCCTTCCGACGCTACAGCCGCACCTTCCCAACCATCAACATTGAGCCT
ProGluGlyGluProPheArgArgTyrSerArgThrPheProThrIleAsnIleGluPro

        550        560        570
GACATCAAGCGCCTCCTTAAAGTTGCCATATAGATGTGAACTGCTCAACACACAGATCTC
AspIleLysArgLeuLeuLysValAlaIle***


CTCATCCATCCAGTCCTGAGGAGCCTTAGGATGCAGCATGCCTTCAGGAGACACTGCTGG


ACCTCAGCATTCCCTTGATATCAGTCCCCTTCACTGCAGAGCCTTGCCTTTCCCCTCTGC


CTGTTTCCTTTTCCTCTCCCAACCCTCTGGTTGGTGATTCAACTTGGGCTCCAAGACTTG


GGTAAGCTCTGGGCCTTCACAGAATGATGGCACCTTCCTAAACCCTCATGGGTGGTGTCT


GAGAGGCGTGAAGGGCCTGGAGCCACTCTGCTAGAAGAGACCAATAAAGGGCAGGTGTGG


AAAAAAAAAAAAAAAAAAAAAAAAAAAAAAAAAA
```

EP 0 347 224 A2

# FIG. 4

E$^P$ : PROMOTER OF EARLY
       GENE IN SV40

Ap : AMPICILLIN RESISTANT

O : ORIGIN OF REPLICATION

# FIG. 5